# EUROPÄISCHE PATENTANMELDUNG

(11) **EP 2 737 881 A1**
(43) Veröffentlichungstag der Anmeldung: **04.06.2014**
(21) Anmeldenummer: 13195153.5
(22) Anmeldetag: 29.11.2013
(51) Int. Cl.: A61F 5/37

(54) **Fixiergurt**

(30) Priorität: 03.12.2012 DE 202012011520 U
(71) Anmelder: Wysozki, Roman, 22763 Hamburg (DE); Sánchez, Alexander, 21266 Jesteburg (DE)
(72) Erfinder: Wysozki, Roman, 22763 Hamburg (DE)
(74) Vertreter: Hauck Patent- und Rechtsanwälte

(57) **Zusammenfassung**

Fixiergurt zur Fixierung eines Patienten auf einem Bett oder einem sonstigen Lager, wobei an den Patienten eine Fixierbandage angelegt ist, die einen Bettgurt, der an dem Bett oder dem sonstigen Lager befestigt ist, und einen Leibgurt aufweist, der die Taille des Patienten umschließt, wobei der Fixiergurt Folgendes aufweist:
• einen zentralen Gurtabschnitt, der in Längsrichtung des Patienten und im Rücken des Patienten angeordnet wird, so dass er den Leibgurt der Fixierbandage kreuzt,
• ein oberes Fixierelement, das ein Herausrutschen des Patienten nach oben aus dem Leibgurt verhindert, mit einem oberen Ende des zentralen Gurtabschnitts verbunden ist und ein freies Ende, das zur Vorderseite des Oberkörpers des Patienten geführt wird, aufweist,
• ein unteres Fixierelement, das ein Herausrutschen des Patienten nach unten aus dem Leibgurt verhindert, mit einem unteren Ende des zentralen Gurtabschnitts verbunden ist und ein freies Ende, dass zur Vorderseite des Oberkörpers des Patienten geführt wird, aufweist, und
• einen Oberkörpergurt, der an dem zentralen Gurtabschnitt befestigt ist, oberhalb des Leibgurts um den Oberkörper des Patienten herumgeführt wird und zwei freie Enden aufweist, wobei
• das freie Ende des oberen Fixierelements und das freie Ende des unteren Fixierelements Verbindungsmittel zur Verbindung dieser beiden freien Enden miteinander aufweisen, und
• die beiden freien Enden des Oberkörpergurts Befestigungsmittel zur Befestigung am oberen Fixierelement und/oder am unteren Fixierelement aufweisen.

## Beschreibung

Die Erfindung betrifft einen Fixiergurt zur Fixierung eines Patienten auf einem Bett oder einem sonstigen Lager, wobei an den Patienten eine Fixierbandage angelegt ist. Die Fixierbandage weist einen Bettgurt und einen Leibgurt auf. Der Bettgurt ist an dem Bett oder dem sonstigen Lager befestigt und der Leibgurt umschließt die Taille des Patienten. Die Fixierbandage wird durch den besonderen Fixiergurt in sinnvoller Weise ergänzt.

Fixierbandagen mit einem Bettgurt und einem Leibgurt sind seit langem bekannt. Häufig ist es erforderlich, die Bewegungsfreiheit des Patienten zusätzlich zu der Fixierung mittels des Leibgurts weiter einzuschränken. Insbesondere gilt es zu verhindern, dass sich der Patient zu weit dreht und infolgedessen teilweise aus dem Bett herausfällt und dass der Patient nach oben oder unten aus dem Leibgurt herausrutscht.

Aus dem Gebrauchsmuster DE 203 17 701 U1 ist eine Fixierbandage mit einem Bettgurt und einem Leibgurt bekannt geworden, die um einen Schultergurt und einen Schrittgurt ergänzt ist. Der Schultergurt verhindert, dass der Patient nach oben aus dem Leibgurt herausrutschen kann. Der Schrittgurt verhindert, dass der Patient nach unten aus dem Leibgurt herausrutschen kann. Bei der bekannten Anordnung werden freie Enden der Schultergurte und ein freies Ende des Schrittgurts getrennt voneinander am Leibgurt der Fixierbandage befestigt. Daher ist es relativ aufwendig, die bekannte Fixierbandage mit den Schultergurten und dem Schrittgurt korrekt anzulegen. Insbesondere in Verbindung mit einer Arbeitsüberlastung des derartige Fixierbandagen einsetzenden Personals kann es beim Anlegen der Fixierbandage zu Fehlern oder Versäumnissen kommen, die unter Umständen eine Gefährdung des Patienten mit sich bringen.

Davon ausgehend ist es die Aufgabe der Erfindung, einen Fixiergurt zur Verwendung in Verbindung mit einer herkömmlichen Fixierbandage zur Verfügung zu stellen, der ein Herausrutschen des Patienten nach oben oder nach unten aus dem Leibgurt zuverlässig verhindert, besonders einfach anzulegen ist und Risiken für den Patienten durch fehlerhafte Anwendung minimiert.

Diese Aufgabe wird gelöst durch den Fixiergurt mit den Merkmalen des Anspruchs 1. Vorteilhafte Ausgestaltungen sind in den sich anschließenden Unteransprüchen angegeben.

Der Fixiergurt dient zur Fixierung eines Patienten auf einem Bett oder einem sonstigen Lager. An den Patienten ist eine Fixierbandage angelegt, die einen Bettgurt, der an dem Bett oder dem sonstigen Lager befestigt ist, und einen Leibgurt aufweist, der die Taille des Patienten umschließt. Der Fixiergurt weist folgendes auf:
- einen zentralen Gurtabschnitt, der in Längsrichtung des Patienten und im Rücken des Patienten angeordnet wird, so dass er den Leibgurt der Fixierbandage kreuzt,
- ein oberes Fixierelement, das ein Herausrutschen des Patienten nach oben aus dem Leibgurt verhindert, mit einem oberen Ende des zentralen Gurtabschnitts verbunden ist und ein freies Ende, das zur Vorderseite des Oberkörpers des Patienten geführt wird, aufweist,
- ein unteres Fixierelement, das ein Herausrutschen des Patienten nach unten aus dem Leibgurt verhindert, mit einem unteren Ende des zentralen Gurtabschnitts verbunden ist und ein freies Ende, dass zur Vorderseite des Oberkörpers des Patienten geführt wird, aufweist, und
- einen Oberkörpergurt, der an dem zentralen Gurtabschnitt befestigt ist, oberhalb des Leibgurts um den Oberkörper des Patienten herumgeführt wird und zwei freie Enden aufweist, wobei
- das freie Ende des oberen Fixierelements und das freie Ende des unteren Fixierelements Verbindungsmittel zur Verbindung dieser beiden freien Enden miteinander aufweisen, und
- die beiden freien Enden des Oberkörpergurts Befestigungsmittel zur Befestigung am oberen Fixierelement und/oder am unteren Fixierelement aufweisen.

Das obere Fixierelement und das untere Fixierelement sind mit dem zentralen Gurtabschnitt verbunden. Sie können hierzu jeweils ein festes Enden aufweisen, das an dem zentralen Gurtabschnitt befestigt ist, oder auch einteilig mit dem zentralen Gurtabschnitt ausgebildet sein. Dadurch kommt es unabhängig von einer etwaigen Befestigung des Fixiergurts an der Fixierbandage zu einer unmittelbaren Krafteinleitung von auf eines der beiden Fixierelemente ausgeübten Kräften in das jeweils andere Fixierelement. Insbesondere wird dadurch eine Gefährdung des Patienten durch das obere Fixierelement verhindert, weil dieses bei einem zu tiefen Hineinrutschen in das untere Fixierelement durch die unmittelbare Verbindung über den zentralen Gurtabschnitt mit nach unten bewegt und insbesondere vom Hals des Patienten ferngehalten wird.

Zudem vereinfacht die Verbindung des oberen und des unteren Fixierelements über einen zentralen Gurtabschnitt die Anwendung des Fixiergurts in Kombination mit einer herkömmlichen Fixierbandage. Hierfür kann der Fixiergurt besonders einfach mit dem zentralen Gurtabschnitt unterhalb oder oberhalb des Leibgurts der Fixierbandage angeordnet werden, insbesondere zwischen dem Leibgurt und einem darunter angeordneten Bettgurt.

Das obere Fixierelement und das untere Fixierelement weisen jeweils ein freies Ende auf, insbesondere jeweils ein einziges freies Ende, d.h. genau ein freies Ende. Mit einem freien Ende ist derjenige Abschnitt des betreffenden Fixierelements gemeint, der von dem mit dem zentralen Gurtabschnitt verbundenen Ende entfernt ist und der sich im Gebrauch der Fixierbandage im Wesentlichen an der Vorderseite des Oberkörpers des Patienten befindet. Mit dem freien Ende ist nicht nur der betreffende Endpunkt des jeweiligen Elements gemeint, sondern ein Abschnitt mit einer gewissen Länge, beispielsweise einer Länge im Bereich von 20 cm bis 60 cm oder darüber hinaus.

Ebenfalls an dem zentralen Gurtabschnitt befestigt ist ein Oberkörpergurt. Dieser wird oberhalb des Leibgurts um den Oberkörper des Patienten herumgeführt und durch Befestigung seiner beiden freien Enden mit den hierfür vorgesehenen Befestigungsmitteln am oberen und/oder unteren Fixierelement befestigt. Auch hier ist mit dem Begriff "freies Ende" ein Gurtabschnitt mit einer gewissen Länge gemeint. Die Befestigungsmittel der freien Enden des Oberkörpergurts können beispielsweise Ösen sein, die auf einen Sockel eines Magnetschlosses aufgesteckt werden. Hierzu kann der Sockel des Magnetschlosses in eine hierfür vorgesehene Öse, gegebenenfalls mit einer Tasche, die einen Teller des Sockels aufnimmt, am oberen bzw. unteren Fixierelement eingesetzt werden. Auf diesen Sockel können beide freien Enden des Oberkörpergurts aufgesteckt werden. In diesem Fall wird nur ein Magnetschloss zum Schließen des Oberkörpergurts benötigt.

Der Oberkörpergurt kann unlösbar an dem zentralen Gurtabschnitt befestigt sein, insbesondere durch Vernähen. Auch eine lösbare Befestigung ist möglich, insbesondere, in dem der Oberkörpergurt durch eine Schlaufe im zentralen Gurtabschnitt oder der zentrale Gurtabschnitt durch eine Schlaufe im Oberkörpergurt hindurchgeführt wird. In diesem Fall kann der Oberkörpergurt einfach ausgetauscht werden.

Der korrekt angelegte Oberkörpergurt stellt den richtigen Sitz des oberen Fixierelements sicher. Insbesondere verhindert der Oberkörpergurt, dass das obere Fixierelement oder ein Teil davon von einer Schulter des Patienten abgestreift werden kann, was zu einer unbequemen oder gefährlichen Situation führen könnte.

Bei der Erfindung weisen das freie Ende des oberen Fixierelements und das freie Ende des unteren Fixierelements Verbindungsmittel zur Verbindung miteinander auf. Die Verbindungsmittel können beispielsweise mehrere Ösen aufweisen, die in den beiden freien Enden angeordnet sind. Mittels dieser Verbindungselemente werden die beiden genannten freien Enden unmittelbar miteinander verbunden, beispielsweise durch Hindurchführen eines Stifts, insbesondere eines Magnetschlosses, durch jeweils eine der Ösen an einem der beiden freien Enden. Es wird also durch das Herstellen dieser einzigen Verbindung ein geschlossener Ring ausgebildet, bestehend aus dem zentralen Gurtabschnitt, dem oberen Fixierelement und dem unteren Fixierelement. Der geschlossene Ring kann demnach ausschließlich aus Elementen des Fixiergurts selbst bestehen. Er kann unabhängig von der Fixierbandage, insbesondere unabhängig von dem Leibgurt, geöffnet und geschlossen werden. Das Anlegen des Fixiergurts ist daher besonders einfach. Insbesondere kann zunächst wie gewohnt der Leibgurt geschlossen werden und erst danach der Fixiergurt. Es ist sogar möglich, die Einstellung des Leibgurts unabhängig von derjenigen des Fixiergurts anzupassen, etwa den Leibgurt nachträglich fester oder lockerer einzustellen, ohne hierzu den Fixiergurt zu öffnen. Dies ist selbst bei unkooperativen Patienten möglich, weil gegebenenfalls in den Fixiergurt eingeleitete Kräfte auch ohne den Leibgurt vom Fixiergurt aufgenommen werden können und das erneute Schließen des Leibgurts nicht erschweren.

Ein besonderer Vorteil ist, dass das Einstellen des oberen Fixierelements und des unteren Fixierelements auf die Körpermaße des Patienten zum selben Zeitpunkt erfolgt, nämlich in dem Augenblick, in dem das Verbindungsmittel geschlossen wird. Dadurch kann auf einfache Weise ein zu lockeres Anlegen des Fixiergurts vermieden werden, weil sichergestellt ist, dass sich der Patient beim Bemessen der Längen der beiden Fixierelemente in exakt derselben Position befindet. Bei der aus dem Stand der Technik bekannten Fixierbandage ist es hingegen möglich, dass sich der Patient beim Befestigen der freien Enden des Schultergurts am Leibgurt in einer ersten Position relativ weit oben befindet, zum Zeitpunkt des Schließens des Schrittgurts jedoch im Bett ein Stück weit nach unten gerutscht ist. Dadurch kann der von den Fixierelementen gebildete Ring unter Umständen zu groß ausfallen.

In einer Ausgestaltung weisen die Verbindungsmittel ein einziges Magnetschloss auf, mit dem die Verbindung zwischen dem freien Ende des oberen Fixierelements und dem freien Ende des unteren Fixierelements gesichert wird und zugleich die beiden freien Enden des Oberkörpergurts befestigt werden. Das Magnetschloss kann einen Sockel mit einem Teller und einem Stift aufweisen. Der Stift kann durch Ösen in allen vier freien Enden hindurchgeführt werden. Dabei kann der Teller des Sockels unterhalb einer Öse des untersten freien Endes angeordnet und dort von einem Gurtstück, das eine Tasche bildet, gehalten sein. Auf das aus der Öse im obersten freien Ende hervorstehende Stiftende kann ein Schließknopf aufgesetzt werden, der nur mit Hilfe eines Magnetschlüssels geöffnet und wieder von dem Sockel entfernt werden kann. Die Verbindung aller vier freien Enden miteinander kann auf diese Weise in einem einzigen Punkt hergestellt oder gesichert werden. Die Verwendung eines einzigen Magnetschlosses hierfür bedeutet eine weitere Vereinfachung beim Anlegen des Fixiergurts sowie zugleich eine Materialersparnis, weil nur ein Magnetschloss benötigt wird.

In einer Ausgestaltung weisen die Verbindungsmittel einen Ring am freien Ende des oberen Fixierelements oder am freien Ende des unteren Fixierelements auf, durch den das andere dieser beiden freien Enden hindurchführbar ist, sowie mehrere Ösen an dem anderen freien Ende. Zum Herstellen der Verbindung zwischen den freien Enden des oberen und unteren Fixierelements wird also das freie Ende mit den Ösen durch den an dem anderen freien Ende angeordneten Ring hindurchgeführt. Es wird dann mit Hilfe der Ösen fixiert, sodass das freie Ende mit den Ösen eine durch den Ring hindurchgeführte Schlaufe bildet.

Zum Befestigen des freien Endes mit den Ösen kann wiederum ein Magnetschloss eingesetzt werden. Wie bei der zuvor beschriebenen Ausgestaltung können mit demselben Magnetschloss zugleich die beiden freien Enden des Oberkörpergurts befestigt werden.

Der Ring kann ein Metallring sein und/oder kann eine rechteckige Form aufweisen. Die Verwendung des Rings bietet mehrere Vorteile. Zum einen vereinfacht sich die Handhabung, weil zum Einstellen der richtigen Länge des Fixiergurts nach dem Hindurchführen des einen freien Endes durch den Ring nur noch dieses eine freie Ende gehandhabt werden muss. Zum anderen führt die schlaufenförmige Anordnung des freien Endes mit den Ösen zu einer deutlichen Entlastung eines für die Befestigung eingesetzten Magnetschlosses. Grund hierfür ist, dass auftretende Zugbelastungen wegen der Umlenkung des freien Endes mit den Ösen durch den Ring weit weniger stark zu einer Zugbelastung auf das Magnetschloss führen, als zwei von entgegengesetzten Richtungen aus am Magnetschloss zusammengeführte freie Enden.

In einer Ausgestaltung ist der Ring am freien Ende des unteren Fixierelements ausgebildet. In diesem Fall weist das freie Ende des oberen Fixierelements die Ösen auf und wird vor der Brust des Patienten geschlossen. In diesem Fall ist eine gleichzeitige Befestigung der freien Enden der Oberkörpergurte im selben Punkt besonders vorteilhaft.

In einer Ausgestaltung weist das freie Ende des oberen Fixierelements an der Brust des Patienten, in einem Abschnitt zwischen dem Hals des Patienten und der Position, an der im Gebrauch der Ring angeordnet ist, eine einzige Öse zur Aufnahme eines Sockels des Magnetschlosses auf. Unterhalb der Öse kann eine Tasche angeordnet sein, die einen Teller des Sockels des Magnetschlosses aufnimmt. Die einzige Öse kann ungefähr auf Höhe der Achseln des Patienten angeordnet sein, insbesondere oberhalb des Sternums. In diesem Fall wird allein durch die Position der einzigen Öse sichergestellt, dass der Oberkörpergurt so weit oben wie möglich angelegt wird. Dadurch wird besonders zuverlässig verhindert, dass der Patient mit einem Arm in den Oberkörpergurt hineingeraten oder das obere Fixierelement ganz oder teilweise abstreifen kann.

In einer Ausgestaltung weist der Fixiergurt Befestigungsmittel zur Befestigung des Fixiergurts an der Fixierbandage auf. Eine Befestigung an der Fixierbandage, entweder am Bettgurt oder am Leibgurt, ist in der Regel notwendig, damit der Fixiergurt die gewünschte Wirkung entfalten kann. Grundsätzlich kann diese Befestigung durch eine dauerhafte Verbindung zwischen Fixierbandage und Fixiergurt hergestellt werden, insbesondere durch Vernähen. Beispielsweise kann der zentrale Gurtabschnitt des Fixiergurts mit dem Leibgurt oder dem Bettgurt der Fixierbandage vernäht sein. In diesem Fall kann eine herkömmliche Fixierbandage mit dem Fixiergurt kombiniert werden. Insbesondere bei einer Fixierbandage, bei der der Leibgurt mit scherenartig angeordneten Gurten am Bettgurt befestigt ist, um ein seitliches Abrollen des Leibgurts auf dem Bettgurt zu ermöglichen, ist eine Befestigung des Fixiergurts am Leibgurt von Vorteil, weil die Möglichkeit des Abrollens erhalten bleibt. Es ist jedoch auch möglich, den zentralen Gurtabschnitt des Fixiergurts zwischen dem Bettgurt und dem Leibgurt anzuordnen und diese drei Lagen alle fest miteinander zu verbinden, insbesondere zu vernähen. Die Verwendung von am Fixiergurt ausgebildeten Befestigungsmitteln kann demgegenüber die Nachrüstung einer herkömmlichen Fixierbandage vereinfachen.

In einer Ausgestaltung sind die Befestigungsmittel zur Befestigung des Fixiergurts an dem zentralen Gurtabschnitt angeordnet. Bei diesen Befestigungsmitteln kann es sich insbesondere um einen Klettverschluss handeln, beispielsweise ein Klett- oder Flauschband, das mit dem zentralen Gurtabschnitt vernäht ist. Das jeweilige Gegenstück, also ein Flausch- oder Klettband, ist an der zugehörigen Fixierbandage angeordnet, insbesondere an einer Unterseite des Leibgurts.

In einer Ausgestaltung sind die Befestigungsmittel zur Befestigung des Fixiergurts von Abschnitten des Oberkörpergurts gebildet, die durch Schlaufen an der Fixierbandage hindurchgeführt werden. Dann müssen zur Befestigung des Fixiergurts an der Fixierbandage lediglich die beiden freien Enden des Oberkörpergurts durch die betreffenden Schlaufen an der Fixierbandage hindurchgezogen werden. In Verbindung mit einer entsprechend ausgebildeten Fixierbandage stellt dies eine besonders einfache Befestigungsmöglichkeit dar.

In einer Ausgestaltung weist der Fixiergurt mehrere Oberkörpergurte auf, die in einem Abstand voneinander am zentralen Gurtabschnitt befestigt sind. Jeder der Oberkörpergurte weist zwei freie Enden auf, die Befestigungsmittel zur Befestigung am oberen Fixierelement und/oder am unteren Fixierelement aufweisen, wie in Verbindung mit dem Oberkörpergurt bereits erläutert. Durch die Verwendung mehrerer Oberkörpergurte kann das obere Fixierelement besonders sicher angelegt werden. Sofern einer der Oberkörpergurte zur Befestigung des Fixiergurts an der Fixierbandage durch Schlaufen an der Fixierbandage hindurchgeführt wird, wie vorstehend erläutert, kann ein weiterer Oberkörpergurt weiter oben, insbesondere nahe unter den Achseln des Patienten, angelegt werden und so ein Abstreifen des oberen Fixierelements wirksam verhindern.

In einer Ausgestaltung weist der zentrale Gurtabschnitt mehrere, auf unterschiedlicher Höhe angeordnete Schlaufen zur Befestigung eines Oberkörpergurts auf. Der mithilfe der Schlaufen befestigte Oberkörpergurt kann dann stets auf optimaler Höhe angelegt werden, indem er durch die auf eine etwa auf dieser Höhe angeordnete Schlaufe hindurchgeführt wird. Bei Verwendung mehrerer Oberkörpergurte kann einer der Oberkörpergurte unlösbar mit dem zentralen Gurtabschnitt verbunden sein, während ein weiterer durch eine der Schlaufen des zentralen Gurtabschnitts befestigt wird. Ebenfalls mit dem Ziel einer optimalen Höhenanpassung kann auch die Befestigung der freien Enden des betreffenden Oberkörpergurts am freien Ende des oberen bzw. des unteren Fixierelements auf unterschiedlicher Höhe erfolgen, insbesondere, indem dort mehrere Aufnahmen für ein Magnetschloss vorgesehen sind. Dann kann die Höhe des Oberkörpergurts oder eines weiteren Oberkörpergurts sowohl im Rücken des Patienten als auch vom individuell gewählt werden.

In einer Ausgestaltung weist das obere Fixierelement zwei über die Schultern des Patienten zu führende Schultergurte auf, die an der Vorderseite des Oberkörpers des Patienten zu dem freien Ende zusammengeführt sind. Insbesondere können die beiden Schultergurte miteinander und/oder mit einem weiteren Gurtabschnitt vernäht sein, sodass sie entweder selbst oder in Verbindung mit dem weiteren Gurtabschnitt das freie Ende bilden. Im Rücken des Patienten können die beiden Schultergurts H-förmig ausgebildet sein, d.h. im Wesentlichen parallel verlaufen und mit einem Querverbindungsstück miteinander verbunden sein. Eine X-förmige, also sich überkreuzende Anordnung im Rücken des Patienten ist ebenfalls möglich. Mit derartigen Schultergurten wird eine zuverlässige Fixierung des Patienten erreicht. Bei der Verwendung eines Schultergurts als oberes Fixierelement können die freien Enden des Oberkörpergurts jeweils an einem Schultergurt befestigt werden. Hierzu können an den freien Enden des Oberkörpergurts zwei oder mehr Aufnahmen für die Sockel von Magnetschlössern ausgebildet sein.

In einer Ausgestaltung weist das obere Fixierelement eine über den Kopf des Patienten zu ziehende Kragenschlaufe auf. Die Kragenschlaufe kann von zwei Gurtabschnitten gebildet sein, die zu beiden Seiten des Halses des Patienten angeordnet sind. Die im Rücken befindlichen Enden dieser Gurtabschnitte sind miteinander verbunden, insbesondere vernäht, gegebenenfalls mit Hilfe eines separaten Verbindungsstücks. Außerdem sind sie dort fest mit dem zentralen Gurtabschnitt verbunden. Auf der Brust des Patienten sind die beiden Gurtabschnitte ebenfalls miteinander verbunden, wahlweise mit einem weiteren Verbindungsstück. Außerdem sind sie mit dem freien Ende des oberen Fixierelements verbunden bzw. bilden dieses. Eine solche Kragenschlaufe verläuft seitlich relativ nah am Hals des Patienten und kann nicht seitlich von einer Schulter abgestreift werden, was bei Schultergurten unter Umständen möglich ist. Sie stellt daher eine besonders sichere Form der Fixierung dar. Gleichzeitig kann sie ein Maximum an Bewegungsfreiheit für die Schultern und Arme des Patienten erhalten.

In einer Ausgestaltung ist das untere Fixierelement ein Schrittgurt. Ein solcher Schrittgurt wird zwischen den Beinen des Patienten hindurchgeführt. Er kann ein Herausrutschen des Patienten aus dem Leibgurt nach unten zuverlässig verhindern.

In einer Ausgestaltung weist das untere Fixierelement zwei Oberschenkelmanschetten auf, die jeweils über einen ersten Gurt mit dem zentralen Gurtabschnitt und über einen zweiten Gurt mit dem freien Ende des unteren Fixierelements verbunden sind. Es versteht sich, dass die beiden zweiten Gurte an ihren von den Oberschenkelmanschetten entfernten Enden miteinander vernäht sein können und so das freie Ende des unteren Fixierelements bilden können. Derartige Oberschenkelmanschetten stellen ebenfalls sicher, dass der Patient nicht nach unten aus dem Leibgurt herausrutscht. Gleichzeitig können sie einen höheren Tragekomfort bieten als ein Schrittgurt.

In einer Ausgestaltung ist der Fixiergurt mit einer Fixierbandage kombiniert, die einen Bettgurt zur Befestigung an dem Bett oder dem sonstigen Lager und einen Leibgurt aufweist, der dazu ausgebildet ist, die Taille des Patienten zu umschließen. In einer Ausgestaltung ist der Fixiergurt unlösbar an der Fixierbandage befestigt, insbesondere durch Vernähen oder Verkleben. Insbesondere kann der zentrale Gurtabschnitt des Fixiergurts mit dem Leibgurt oder mit dem Bettgurt der Fixierbandage verbunden sein. Im Vergleich zu einer lösbaren Befestigung mittels Schlaufen am Leib- oder Bettgurt bietet diese Lösung den Vorteil eines frei wählbaren Abstands des Oberkörpergurts von der Fixierbandage.

Nachfolgend wird die Erfindung anhand eines in Figuren dargestellten Ausführungsbeispiels näher erläutert. Es zeigen:
- Fig. 1: einen erfindungsgemäßen Fixiergurt in einer ausgebreiteten Anordnung,
- Fig. 2: den Fixiergurt aus Fig. 1 im geschlossenen Zustand,
- Fig. 3: den Fixiergurt aus Fig. 1 in ausgebreiteter Anordnung in Verbindung mit einer Fixierbandage, und
- Fig. 4: den Fixiergurt und die Fixierbandage aus Fig. 3 im geschlossenen Zustand.

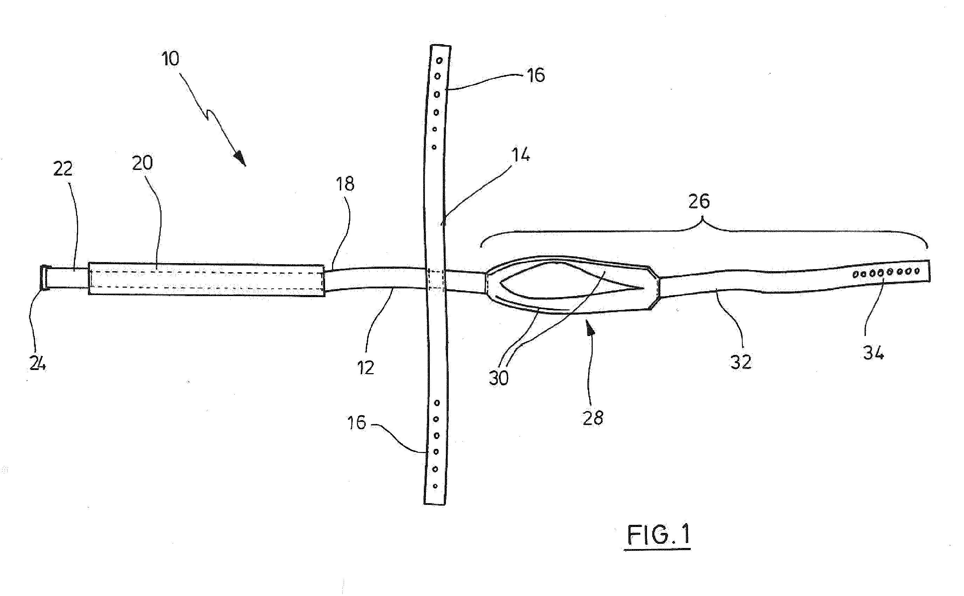

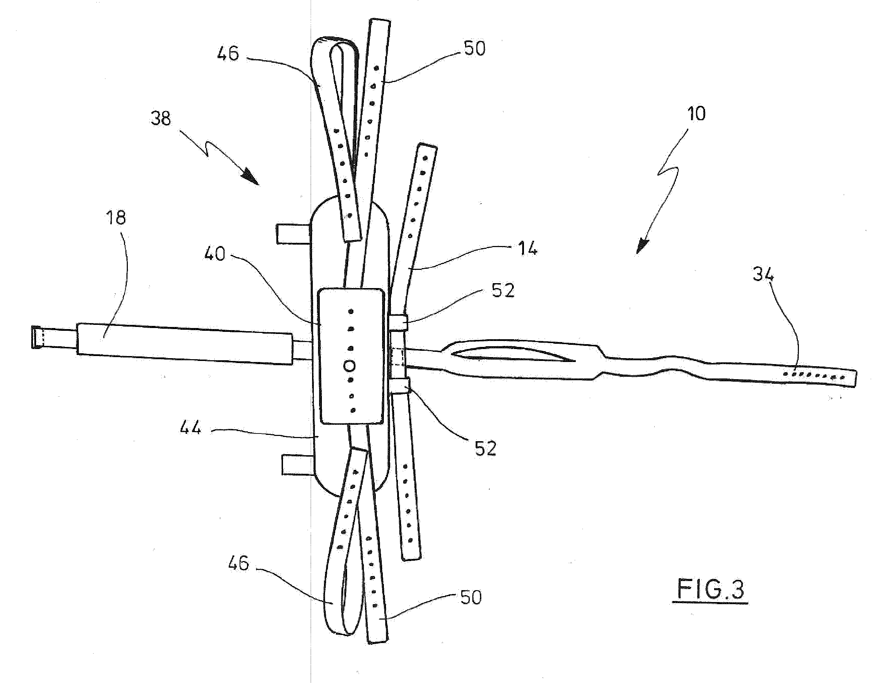

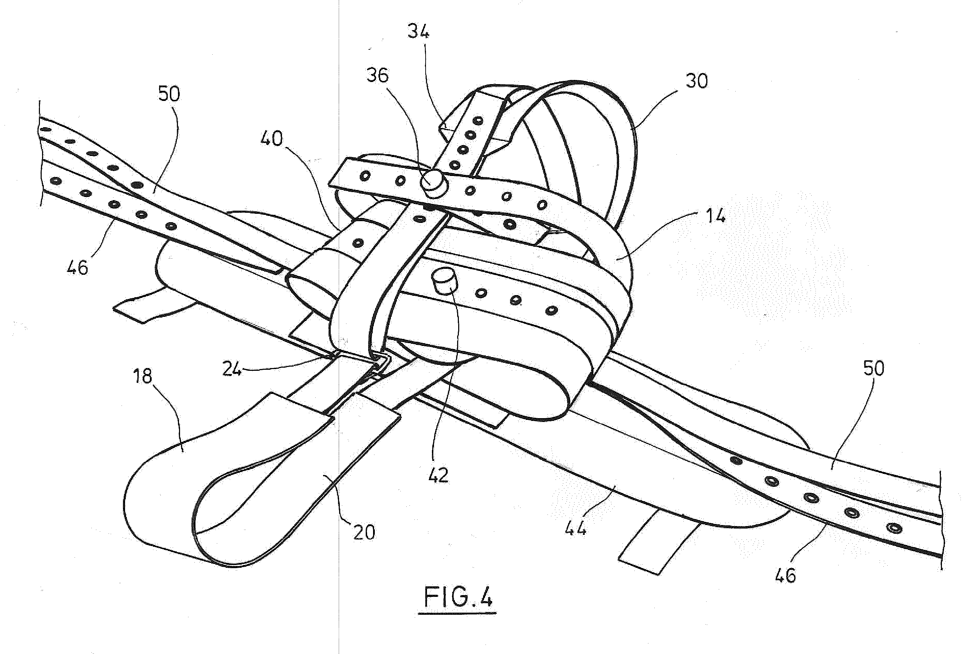

Der Fixiergurt 10 aus Fig. 1 weist in der Mitte einen zentralen Gurtabschnitt 12 auf, der sich im Gebrauch des Fixiergurts im Rücken des Patienten befindet, wobei er in Längsrichtung des Rückens verläuft und den Leibgurt 40 einer Fixierbandage 38 (siehe hierzu Figuren 3 und 4) kreuzt.

Am zentralen Gurtabschnitt 12 ist ein Oberkörpergurt 14 befestigt. Hierzu ist im gezeigten Ausführungsbeispiel die Mitte des Oberkörpergurts 14 mit einem mittleren Abschnitt des zentralen Gurtabschnitts 12 vernäht. Der Oberkörpergurt 14 ist im rechten Winkel zum zentralen Gurtabschnitt 12 angeordnet. Der Oberkörpergurt 14 weist zwei freie Enden 16 auf. Jedes der freien Enden 16 weist eine Vielzahl von Ösen auf, die in gleichmäßigen Abschnitten voneinander angeordnet sind.

Das untere, d.h. zu den Füßen des Patienten hin weisende Ende des zentralen Gurtabschnitts 12 ist mit einem Schrittgurt 18 verbunden bzw. geht bei einteiliger Fertigung in diesen Schrittgurt 18 über. Der Schrittgurt 18 weist ein Polster 20 auf, das im Schritt des Patienten angeordnet wird. Außerdem hat der Schrittgurt 18 ein freies Ende 22, an dem ein rechteckiger Ring 24 aus Metall befestigt ist.

Mit dem oberen Ende des zentralen Gurtabschnitts 12, d.h. mit dem zum Kopf des Patienten hin weisenden Ende, ist ein oberes Fixierelement 26 verbunden, das eine Kragenschlaufe 28 aufweist. Die Kragenschlaufe 28 weist zwei benachbart, zu beiden Seiten des Halses des Patienten angeordnete Gurtabschnitte 30 auf, die gepolstert sind und die einen geschlossenen Ring bilden. Die in der Figur 1 links gezeigten Enden dieser Gurtabschnitte 30 sind mit dem oberen Ende des zentralen Gurtabschnitts 12 verbunden, auch hier durch Vernähen. Die in der Figur 1 rechts angeordneten Enden der beiden Gurtabschnitte 30 sind ebenfalls miteinander verbunden sowie mit einem weiteren Gurtabschnitt 32, der ein freies Ende 34 des oberen Fixierelements 26 bildet. Das freie Ende 34 des oberen Fixierelements 26 weist eine Vielzahl von Ösen auf.

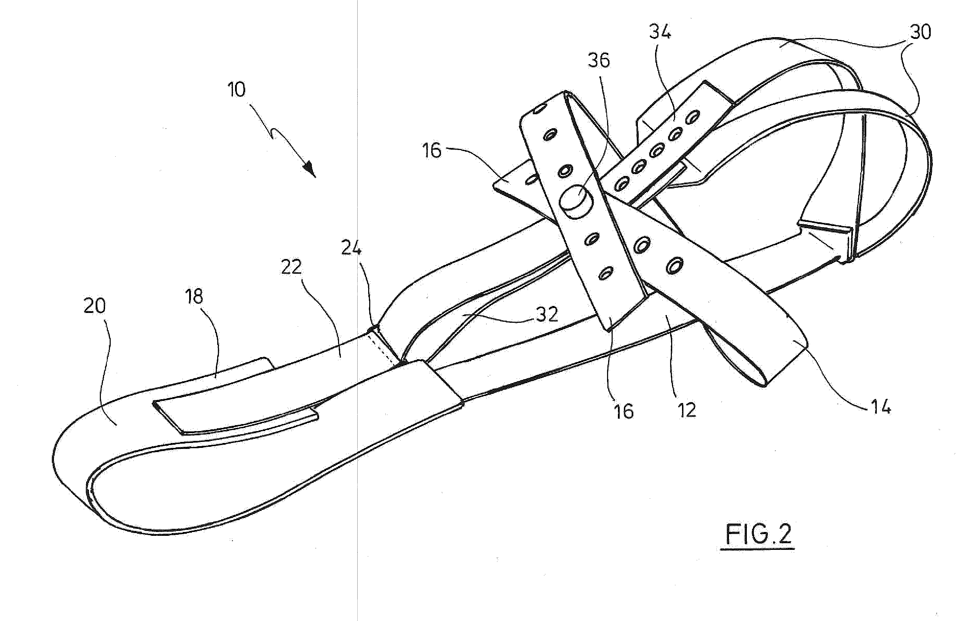

Fig. 2 zeigt den geschlossenen Zustand des Fixiergurts 10 aus Fig. 1. Man erkennt den im Rücken des Patienten anzuordnenden zentralen Gurtabschnitt 12 und den um den Oberkörper des Patienten herumgeführten Oberkörpergurt 14, der mit dem zentralen Gurtabschnitt 12 im Rücken des Patienten verbunden ist. Die beiden freien Enden 16 des Oberkörpergurts 14 sind an dem freien Ende 34 des oberen Fixierelements 26 befestigt, und zwar mit Hilfe eines Magnetschlosses, von dem in der Figur 2 der Schließknopf 36 zu erkennen ist.

Der Schrittgurt 18 ist umgeschlagen und wird an der Vorderseite des Oberkörpers des Patienten nach oben geführt, sodass der Ring 24 auf dem Bauch des Patienten angeordnet ist.

Man erkennt in der Fig. 2, dass das freie Ende 34 des oberen Fixierelements 26 von unten durch den Ring 24 hindurchgeführt und nachfolgend auf zur Brust des Patienten hin umgeschlagen ist. In dem zur Kragenschlaufe 28 weisenden Teil des weiteren Gurtabschnitts 32 ist eine einzige Öse mit einer Tasche ausgebildet, die in der Figur verdeckt ist und die einen ebenfalls verdeckten Sockel des Magnetschlosses aufnimmt. Diese einzige Öse nebst Tasche und damit auch das Magnetschloss befinden sich relativ nahe an der Kragenschlaufe 28. Das freie Ende 34 des oberen Fixierelements 26 ist mit einer Öse auf einen Stift des Magnetschlosses aufgesteckt. Darüber befinden sich die ebenfalls mit Hilfe jeweils einer Öse aufgesteckten freien Enden 16 des Oberkörpergurts 14. Die gesamte Anordnung wird durch den Schließknopf 36 gesichert.

Figur 3 zeigt den Fixiergurt 10 auf Fig. 1 nochmals im ausgebreiteten Zustand in Kombination mit einer Fixierbandage 38. Die Fixierbandage 38 weist einen Leibgurt 40 auf, der mit einer Vielzahl von Ösen versehen ist und mit Hilfe eines weiteren Magnetschlosses 42 um die Taille des Patienten herum geschlossen wird. Außerdem umfasst die Fixierbandage 38 einen Bettgurt 44, der flach auf eine Matratze aufgelegt und mit Hilfe zweier Betthalterungen 46, die ebenfalls eine Vielzahl von Ösen aufweisen, beispielsweise an Holmen des Betts befestigt wird. Der Leibgurt 40 ist mit dem Bettgurt 44 verbunden.

Seitlich am Leibgurt 40 sind zwei Seitenbefestigungen 50 befestigt, die ebenfalls am Bettgestell fixiert werden können, um ein übermäßiges Drehen des Patienten zu verhindern.

Der Leibgurt 40 weist außerdem an seinem oberen Rand, also dem zum Kopf des Patienten weisenden Rand, zwei im Rücken des Patienten angeordnete Schlaufen 52 auf. Durch diese Schlaufen 52 ist der Oberkörpergurt 14 hindurchgeführt. Der zentrale Abschnitt 12 des Fixiergurts 10 verläuft zwischen den beiden Schlaufen 52 im Rücken des Patienten sowie zwischen der Unterseite des Leibgurts 40 und dem Bettgurt 44.

Fig. 4 zeigt den vollständig geschlossenen Zustand der Anordnung aus Fig. 3. Man erkennt, dass der Oberkörpergurt 14 oberhalb des Leibgurts 40 um den Oberkörper des Patienten herum geschlossen ist. Ebenfalls gut erkennbar ist, dass der Schrittgurt 18 als unteres Fixierelement über den Ring 24 unmittelbar mit dem oberen Fixierelement 26 verbunden ist, und zwar völlig unabhängig von dem Leibgurt 40.

## Patentansprüche

1. Fixiergurt (10) zur Fixierung eines Patienten auf einem Bett oder einem sonstigen Lager, wobei an den Patienten eine Fixierbandage (38) angelegt ist, die einen Bettgurt (44), der an dem Bett oder dem sonstigen Lager befestigt ist, und einen Leibgurt (40) aufweist, der die Taille des Patienten umschließt, wobei der Fixiergurt (10) Folgendes aufweist:
• einen zentralen Gurtabschnitt (12), der in Längsrichtung des Patienten und im Rücken des Patienten angeordnet wird, so dass er den Leibgurt (40) der Fixierbandage (38) kreuzt,
• ein oberes Fixierelement (26), das ein Herausrutschen des Patienten nach oben aus dem Leibgurt (40) verhindert, mit einem oberen Ende des zentralen Gurtabschnitts (12) verbunden ist und ein freies Ende (34), das zur Vorderseite des Oberkörpers des Patienten geführt wird, aufweist,
• ein unteres Fixierelement, das ein Herausrutschen des Patienten nach unten aus dem Leibgurt (40) verhindert, mit einem unteren Ende des zentralen Gurtabschnitts (12) verbunden ist und ein freies Ende (22), dass zur Vorderseite des Oberkörpers des Patienten geführt wird, aufweist, und
• einen Oberkörpergurt (14), der an dem zentralen Gurtabschnitt (12) befestigt ist, oberhalb des Leibgurts (40) um den Oberkörper des Patienten herumgeführt wird und zwei freie Enden (16) aufweist, wobei
• das freie Ende (34) des oberen Fixierelements (26) und das freie Ende (22) des unteren Fixierelements Verbindungsmittel zur Verbindung dieser beiden freien Enden (22, 34) miteinander aufweisen, und
• die beiden freien Enden (16) des Oberkörpergurts (14) Befestigungsmittel zur Befestigung am oberen Fixierelement und/oder am unteren Fixierelement aufweisen.

2. Fixiergurt (10) nach Anspruch 1, **dadurch gekennzeichnet, dass** die Verbindungsmittel ein einziges Magnetschloss aufweisen, mit dem die Verbindung zwischen dem freien Ende (34) des oberen Fixierelements (26) und dem freien Ende (22) des unteren Fixierelements gesichert wird und zugleich die beiden freien Enden (16) des Oberkörpergurts (14) befestigt werden.

3. Fixiergurt (10) nach Anspruch 1 oder 2, **dadurch gekennzeichnet, dass** die Verbindungsmittel einen Ring (24) am freien Ende (34) des oberen Fixierelements (26) oder am freien Ende (22) des unteren Fixierelements aufweisen, durch den das andere dieser beiden freien Enden (22, 34) hindurchführbar ist, sowie mehrere Ösen an dem anderen freien Ende (22, 34).

4. Fixiergurt (10) nach Anspruch 3, **dadurch gekennzeichnet, dass** der Ring (24) am freien Ende (22) des unteren Fixierelements ausgebildet ist.

5. Fixiergurt (10) nach Anspruch 4, **dadurch gekennzeichnet, dass** das freie Ende (34) des oberen Fixierelements (26) an der Brust des Patienten, in einem Abschnitt zwischen dem Hals des Patienten und der Position, an der im Gebrauch der Ring (24) angeordnet ist, eine einzige Öse zur Aufnahme eines Sockels des Magnetschlosses aufweist.

6. Fixiergurt (10) nach einem der Ansprüche 1 bis 5, **dadurch gekennzeichnet**" dass der Fixiergurt (10) Befestigungsmittel zur Befestigung des Fixiergurts (10) an der Fixierbandage (38) aufweist.

7. Fixiergurt (10) nach Anspruch 6, **dadurch gekennzeichnet, dass** die Befestigungsmittel zur Befestigung des Fixiergurts (10) an dem zentralen Gurtabschnitt (12) angeordnet sind.

8. Fixiergurt (10) nach Anspruch 6 oder 7, **dadurch gekennzeichnet, dass** die Befestigungsmittel zur Befestigung des Fixiergurts (10) von Abschnitten des Oberkörpergurts (14) gebildet sind, die durch Schlaufen (52) an der Fixierbandage (38) hindurchgeführt werden.

9. Fixiergurt (10) nach einem der Ansprüche 1 bis 8, **dadurch gekennzeichnet, dass** der Fixiergurt mehrere Oberkörpergurte aufweist, die in einem Abstand voneinander am zentralen Gurtabschnitt (12) befestigt sind.

10. Fixiergurt (10) nach einem der Ansprüche 1 bis 9, **dadurch gekennzeichnet, dass** der zentrale Gurtabschnitt (12) mehrere, auf unterschiedlicher Höhe angeordnete Schlaufen zur Befestigung eines Oberkörpergurts aufweist.

11. Fixiergurt (10) nach einem der Ansprüche 1 bis 10, **dadurch gekennzeichnet, dass** das obere Fixierelement (26) zwei über die Schultern des Patienten zu führende Schultergurte aufweist, die an der Vorderseite des Oberkörpers des Patienten zu dem freien Ende (34) zusammengeführt sind.

12. Fixiergurt (10) nach einem der Ansprüche 1 bis 11, **dadurch gekennzeichnet, dass** das obere Fixierelement (26) eine über den Kopf des Patienten zu ziehende Kragenschlaufe (28) aufweist.

13. Fixiergurt (10) nach einem der Ansprüche 1 bis 12, **dadurch gekennzeichnet, dass** das untere Fixierelement ein Schrittgurt (18) ist.

14. Fixiergurt (10) nach einem der Ansprüche 1 bis 12, **dadurch gekennzeichnet, dass** das untere Fixierelement zwei Oberschenkelmanschetten aufweist, die jeweils über einen ersten Gurt mit dem zentralen Gurtabschnitt und über einen zweiten Gurt mit dem freien Ende des unteren Fixierelements verbunden sind.

15. Fixiergurt (10) nach einem der Ansprüche 1 bis 14 mit einer Fixierbandage (38), die einen Bettgurt (44) zur Befestigung an dem Bett oder dem sonstigen Lager und einen Leibgurt (40) aufweist, der dazu ausgebildet ist, die Taille des Patienten zu umschließen.

16. Fixiergurt (10) nach Anspruch 15, **dadurch gekennzeichnet, dass** der Fixiergurt (10) unlösbar an der Fixierbandage (38) befestigt ist.
